# EUROPEAN PATENT APPLICATION

(11) **EP 1 815 855 A1**
(43) Date of publication of application: **08.08.2007**
(21) Application number: 07105991.9
(22) Date of filing: 12.11.1999
(51) Int. Cl.: A61K 31/353, A61K 31/4439, A61K 9/32, A61K 9/52

(54) **Pharmaceutical composition for modified release insulin sensitiser**

(30) Priority: 12.11.1998 GB 9824870; 25.05.1999 GB 9912189
(62) Divisional of application: 99956029.5
(71) Applicant: SMITHKLINE BEECHAM PLC, Brentford, Middlesex TW8 9GS (GB); SMITHKLINE BEECHAM CORPORATION, Philadelphia, PA 19101 (US)
(72) Inventor: Glinecke, Robert, King of Prussia, 19406 (US); Milosovich, Susan Marie, Collegeville, PA 19426-0989 (US); Muldoon, William, King of Prussia, PA 19406 (US); Re, Vincenzo, Harlow, Essex CM19 5AW (GB); Sauer, Joseph, King of Prussia, PA 19406 (US); Skinner, Janet Louise, Harlow, Essex CM19 5AW (GB)
(74) Representative: Griffith, Johanna Elise

(57) **Abstract**

A pharmaceutical composition which composition comprises an insulin sensitiser and a pharmaceutically acceptable carrier therefore, wherein the composition is arranged to provide a modified release of the insulin sensitiser and the use of such composition in medicine.

## Description

This invention relates to a novel composition, in particular to a modified release composition and its use in medicine, especially its use for the treatment of diabetes mellitus, preferably Type 2 diabetes, and conditions associated with diabetes mellitus.

European Patent Application, Publication Number 0,306,228 relates to certain thiazolidinedione derivatives disclosed as having antihyperglycaemic and hypolipidaemic activity. One particular thiazolidinedione disclosed in EP 0306228 is 5-[4-[2-(N-methyl-N-(2-pyridyl)amino)ethoxy]benzyl]thiazolidine-2,4-dione (hereinafter 'Compound (I)'). WO94/05659 discloses certain salts of Compound (I) including the maleate salt at example 1 thereof.

Compound (I) is an example of a class of anti-hyperglycaemic agents known as 'insulin sensitisers'. In particular Compound (I) is a thiazolidinedione insulin sensitiser.

European Patent Applications, Publication Numbers: 0008203, 0139421, 0032128, 0428312, 0489663, 0155845, 0257781, 0208420, 0177353, 0319189, 0332331, 0332332, 0528734, 0508740; International Patent Application, Publication Numbers 92/18501, 93/02079, 93/22445 and United States Patent Numbers 5104888 and 5478852, also disclose certain thiazolidinedione insulin sensitisers.

Another series of compounds generally recognised as having insulin sensitiser activity are those typified by the compounds disclosed in International Patent Applications, Publication Numbers WO93/21166 and WO94/01420. These compounds are herein referred to as 'acyclic insulin sensitisers'. Other examples of acyclic insulin sensitisers are those disclosed in United States Patent Number 5232945 and International Patent Applications, Publication Numbers WO92/03425 and WO91/19702.

Examples of other insulin sensitisers are those disclosed in European Patent Application, Publication Number 0533933, Japanese Patent Application Publication Number 05271204 and United States Patent Number 5264451.

The above mentioned publications are incorporated herein by reference.

It is now indicated that a certain modified release pharmaceutical composition containing Compound (I) allows administration of a single daily dose to maintain a sustained advantageous and beneficial effect upon glycaemic control with mimimal expected adverse side effects. Such a formulation is therefore expected to be particularly useful for the treatment of diabetes mellitus, especially Type 2 diabetes and conditions associated with diabetes mellitus.

Accordingly, the invention provides a pharmaceutical composition, suitably for the treatment of diabetes mellitus, especially Type 2 diabetes and conditions associated with diabetes mellitus in a mammal, such as a human, which composition comprises an insulin sensitiser, such as Compound (I) and a pharmaceutically acceptable carrier therefor, wherein the composition is arranged to provide a modified release of the insulin sensitiser.

In a further aspect, the invention provides a modified release pharmaceutical composition, suitably for the treatment of diabetes mellitus, especially Type 2 diabetes and conditions associated with diabetes mellitus in a mammal, such as a human, which composition comprises an insulin sensitiser, such as Compound (I) and a pharmaceutically acceptable carrier therefor, wherein the carrier is arranged to provide a modified release of the insulin sensitiser.

Suitably, the modified release is delayed, pulsed or sustained release.

In one aspect the modified release is a delayed release.

Delayed release is conveniently obtained by use of a gastric resistant formulation such as an enteric formulation, such as a tablet or multi-particulates, such as multiparticulate spheres, coated with a gastric resists, polymer, including Eudragit L100-55, for example as Eudragit L30D-55or Eudragit FS 30D Other gastric resistant polymers include methacrylates, cellulose acetate phthalate, polyvinyl acetate phthalate, hydroxypropyl methylcellulose phtahlate, in particular, Aquateric, Sureteric, HPMCP-HP-55S.

The enteric coated tablet may be a single layer tablet or a multi-layer tablet, such as a bi-or tri- layer tablet, wherein the active agent is present in one or more discrete layers within the compressed tablet form. The discrete tablet layers can be arranged as required to provide modified or non-modified release of active agent.

The multiparticulates include coated drug-coated non-pareil substrates, such as lactose spheres, or drug containing non-pareil substrates, such as drug containing lactose spheres. Such multiparticulates are coated as required with the appropriate enteric formulation, such as Eudragit L100-55, for example as Eudragit L30D-55or Eudragit FS 30D.

In a further aspect the modified release is a sustained release, for example providing effective release of the active agent over a time period of up to 26 hours, typically in the range of 4 to 24 hours.

Sustained release is typically provided by use of a sustained release matrix, usually in tablet form, such as disintegrating, non-disintegrating or eroding matrices.

Sustained release is suitably obtained by use of a non-disintegrating matrix tablet formulation, for example by incorporating Eudragit RS into the tablet. Alternative non disintegrating matrix tablet formulations are provided by incorporating methacrylates, cellulose acetates, hydroxypropyl methylcellulose phtahlate, in particular Eudragit L and RL, Carbopol 971P, HPMCP-HP-55S into the tablet.

Sustained release is further obtained by use of a disintegrating matrix tablet formulation, for example by incorporating methacrylates, methylcellulose, in particular Eudragit L, Methocel K4M into the tablet.

Sustained release can also be achieved by using a semi-permeable membrane coated tablet for example by applying methacrylates, ethylcellulose, cellulose acetate, in particular Eudragit RS, Surelease to the tablet.

Sustained release can also be achieved by using a multi layer tablet, where the active ingredient is formulated differently in each layer.

Sustained release can also be achieved by using multiparticulates coated with semipermeable membranes. The multiparticulates include coated drug-coated non-pareil substrates, such as lactose spheres, or drug containing substrates, such as drug containing lactose/Avicel spheres. Such multiparticulates are coated as required with the appropriate semi-permeable membranes, such as ethylcellulose polymer

In yet a further aspect the modified release is a pulsed release, for example providing up to 4, for example 2, pulses of active agent per 24 hours.

One form of pulsed release is a combination of non-modified release of active agent and delayed release.

Suitable modified release includes controlled release. The composition of the invention also envisages a combination of pulsed, delayed and/or sustained release for the active agent, thereby enabling the release of the reagent at different times. One example of such combination provides non-modified and sustained release of active agent, for example non-modified release of 2mg of Compound (I) and sustained release of 8mg of Compound (I) over a 12 hour period or non-modified release of 2mg of Compound (I) and sustained release of 6mg of Compound (I) over a 12 hour period or non-modified release of 1mg of Compound (I) and sustained release of 7mg of Compound (I) over an 18 hour period. Such controlled release can be achieved by use of appropriate tablet formulations, such as a multi-layered tablet, or of an appropriate multiparticulate formulation containing a combination of components with appropriate release characteristics.

A preferred thiazolidinedione insulin sensitiser is Compound (I).

Other suitable thiazolidinedione insulin sensitisers include (+) -5-[[4-[(3,4-dihydro-6-hydroxy-2,5,7,8-tetramethyl-2H-1-benzopyran-2-yl)methoxy]phenyl]methyl]-2,4-thiazolidinedione (or troglitazone), 5-[4-[(1-methylcyclohexyl)methoxy]benzyl] thiazolidine-2,4-dione (or ciglitazone), 5-[4-[2-(5-ethylpyridin-2-yl)ethoxy]benzyl] thiazolidine-2,4-dione (or pioglitazone) or 5-[(2-benzyl-2,3-dihydrobenzopyran)-5-ylmethyl)thiazolidine-2,4-dione (or englitazone).

A particular thiazolidinedione insulin sensitiser is 5-[4-[2-(5-ethylpyridin-2-yl)ethoxy]benzyl] thiazolidine-2,4-dione (or pioglitazone).

A particular thiazolidinedione insulin sensitiser is (+) -5-[[4-[(3,4-dihydro-6-hydroxy-2,5,7,8-tetramethyl-2H-1-benzopyran-2-yl)methoxy]phenyl]methyl]-2,4-thiazolidinedione (or troglitazone).

In one particular aspect, the composition comprises 2 to 12 mg of Compound (I).

Suitably the composition comprises 2, 3, 4, 5, 6, 7, 8, 9, 10, 11 or 12 mg of Compound (I).

Particularly, the composition comprises 2 to 4 , 4 to 8 or 8 to 12 mg of Compound (I).

Particularly, the composition comprises 2 to 4mg of Compound (I).

Particularly, the composition comprises 4 to 8mg of Compound (I).

Particularly, the composition comprises 8 to 12 mg of Compound (I).

Preferably, the composition comprises 2 mg of Compound (I).

Preferably, the composition comprises 4 mg of Compound (I).

Preferably, the composition comprises 8 mg of Compound (I).

Suitable amounts of the insulin sensitisers include the known permissible doses for these compounds as described or referred to in reference texts such as the British and US Pharmacopoeias, Remington's Pharmaceutical Sciences (Mack Publishing Co.), Martindale The Extra Pharmacopoeia (London, The Pharmaceutical Press) (for example see the 3 1 st Edition page 341 and pages cited therein) or the above mentioned publications.

Suitable unit dosages of other insulin sensitisers include from 100 to 800mg of troglitazone such as 200, 400, 600 or 800mg or from 5 to 50mg, including 10 to 40mg, of pioglitazone, such as 20, 30 or 40 mg and also including 15, 30 and 45mg of pioglitazone.

It will be understood that the insulin sensitiser, such as Compound (I) is administered in a pharmaceutically acceptable form, including pharmaceutically acceptable derivatives such as pharmaceutically acceptable salts, esters and solvates thereof, as appropriate of the relevant pharmaceutically active agent.

It will be understood that all pharmaceutically acceptable forms of the active agents per se are encompassed by this invention. Suitable pharmaceutically acceptable forms of insulin sensitisers include those described in the above mentioned publications.

Suitable pharmaceutically acceptable forms of Compound (I) include those described in EP 0306228 and WO94/05659, especially pharmaceutically acceptable salted forms. A preferred pharmaceutically acceptable salt is a maleate.

Suitable pharmaceutically acceptable solvated forms of Compound (I) include those described in EP 0306228 and WO94/05659, in particular hydrates.

Suitable pharmaceutically acceptable forms of the insulin sensitiser depend upon the particular agent used but includes known pharmaceutically acceptable forms of the particular compound chosen. Such derivatives are found or are referred to in standard reference texts such as the British and US Pharmacopoeias, Remington's Pharmaceutical Sciences (Mack Publishing Co.), The Extra Pharmacopoeia (London, The Pharmaceutical Press) (for example see the 31st Edition page 341 and pages cited therein).

The insulin sensitiser, such as Compound (I), may exist in one of several tautomeric forms, all of which are encompassed by the invention either as individual tautomeric forms or as mixtures thereof. Where the insulin sensitiser, such as Compound (I), contains one or more chiral carbon atoms, and hence can exist in two or more stereoisomeric forms, all of these isomeric forms whether as individual isomers or as mixtures of isomers, including racemates are encompassed by the invention.

The insulin sensitiser of choice is prepared according to known methods, such methods are found or are referred to in standard reference texts, such as the British and US Pharmacopoeias, Remington's Pharmaceutical Sciences (Mack Publishing Co.), Martindale The Extra Pharmacopoeia (London, The Pharmaceutical Press) (for example see the 31st Edition page 341 and pages cited therein) or as described in the above mentioned publications.

For example, Compound (I) or, a pharmaceutically acceptable salt thereof, or a pharmaceutically acceptable solvate thereof, may be prepared using known methods, for example those disclosed in EP 0306228 and WO94/05659. The disclosures of EP 0306228 and WO94/05659 are incorporated herein by reference.

When used herein the term 'conditions associated with diabetes' includes those conditions associated with the pre-diabetic state, conditions associated with diabetes mellitus itself and complications associated with diabetes mellitus.

When used herein the term 'conditions associated with the pre-diabetic state' includes conditions such as insulin resistance, including hereditary insulin resistance, impaired glucose tolerance and hyperinsulinaemia.

'Conditions associated with diabetes mellitus itself include hyperglycaemia, insulin resistance, including acquired insulin resistance and obesity. Further conditions associated with diabetes mellitus itself include hypertension and cardiovascular disease, especially atherosclerosis and conditions associated with insulin resistance. Conditions associated with insulin resistance include polycystic ovarian syndrome and steroid induced insulin resistance and gestational diabetes.

'Complications associated with diabetes mellitus' includes renal disease, especially renal disease associated with Type 2 diabetes, neuropathy and retinopathy.

Renal diseases associated with Type 2 diabetes include nephropathy, glomerulonephritis, glomerular sclerosis, nephrotic syndrome, hypertensive nephrosclerosis and end stage renal disease.

As used herein the term 'pharmaceutically acceptable' embraces both human and veterinary use: for example the term 'pharmaceutically acceptable' embraces a veterinarily acceptable compound.

For the avoidance of doubt, when reference is made herein to scalar amounts, including mg amounts, of Compound (I) in a pharmaceutically acceptable form, the scalar amount referred to is made in respect of Compound (I) per se: For example 2 mg of Compound (I) in the form of the maleate salt is that amount of maleate salt which contains 2 mg of Compound (I).

Diabetes mellitus is preferably Type 2 diabetes.

Glycaemic control may be characterised using conventional methods, for example by measurement of a typically used index of glycaemic control such as fasting plasma glucose or glycosylated haemoglobin (Hb A1c). Such indices are determined using standard methodology, for example those described in: Tuescher A, Richterich, P., Schweiz. med. Wschr. 101 (1971), 345 and 390 and Frank P., 'Monitoring the Diabetic Patent with Glycosolated Hemoglobin Measurements', Clinical Products 1988.

Preferably the treatment of the invention will effect an improvement, relative to the non-modified release of the individual agents, in the levels of advanced glycosylation end products (AGEs), leptin and serum lipids including total cholesterol, HDL-cholesterol, LDL-cholesterol including improvements in the ratios thereof, in particular an improvement in serum lipids including total cholesterol, HDL-cholesterol, LDL-cholesterol including improvements in the ratios thereof.

Usually the compositions are adapted for oral administration. However, they may be adapted for other modes of administration, for example parenteral administration, sublingual or transdermal administration.

In a further aspect the invention also provides a process for preparing a pharmaceutical composition, suitably for the treatment of diabetes mellitus, especially Type 2 diabetes and conditions associated with diabetes mellitus in a mammal, such as a human, which composition comprises all insulin sensitiser, such as Compound (I), and a pharmaceutically acceptable carrier therefor, which process comprises formulating the insulin sensitiser and the pharmaceutically acceptable carrier so as to enable a modified release of the insulin sensitiser.

In a further aspect, the invention provides a process for preparing a modified release pharmaceutical composition, suitably for the treatment of diabetes mellitus, especially Type 2 diabetes and conditions associated with diabetes mellitus in a mammal, such as a human, which composition comprises an insulin sensitiser, such as Compound (I) and a pharmaceutically acceptable carrier therefor, which process comprises formulating the insulin sensitiser and the pharmaceutically acceptable carrier so as to enable a modified release of the insulin sensitiser.

The compositions are formulated to provide the modified release of active agent according to the appropriate methods disclosed in Sustained and Controlled Release Drug Delivery Systems, Editor Joe R Robinson, Volume 7, published by Marcel Dekker under the title Drugs and the Pharmaceutical Sciences, Controlled Drug Delivery, 2nd Edition' edited by Joe Robinson and Vince Lee, Marcel Dekker, 1987 and 'Drug Delivery to the Gastrointestinal Tract' Editors: J G Hardy, S S. Davis and C G Wilson also with reference to texts such as the British and US Pharmacopoeias, Remington's Pharmaceutical Sciences (Mack Publishing Co.), Martindale The Extra Pharmacopoeia (London, The Pharmaceutical Press) (for example see the 31st Edition page 341 and pages cited therein) and Harry's Cosmeticology (Leonard Hill Books).

Preferably, the compositions are in unit dosage form. Unit dosage presentation forms for oral administration may be in tablet or capsule form and may as necessary contain conventional excipients such as binding agents, fillers, lubricants, glidants, disintegrants and wetting agents.

Examples of binding agents include acacia, alginic acid, carboxymethylcellulose calcium, carboxymethylcellulose sodium, dextrates, dextrin, dextrose, ethylcellulose, gelatin, liquid glucose, guar gum, hydroxyethyl cellulose, hydroxypropyl cellulose, hydroxypropyl methylcellulose, magnesium aluminium silicate, maltodextrin, methyl cellulose, polymethacrylates, polyvinylpyrrolidone, pregelatinised starch, sodium alginate, sorbitol, starch, syrup, tragacanth.

Examples of fillers include calcium carbonate, calcium phosphate, calcium sulphate, carboxymethylcellulose calcium, carboxymethylcellulose sodium, compressible sugar, confectioner's sugar, dextrates, dextrin, dextrose, dibasic calcium phosphate dihydrate, dibasic calcium phosphate, fructose, glyceryl palmitostearate, glycine, hydrogenated vegetable oil-type 1, kaolin, lactose, maize starch, magnesium carbonate, magnesium oxide, maltodextrin, mannitol, microcrystalline cellulose, polymethacrylates, potassium chloride, powdered cellulose, pregelatinised starch, sodium chloride, sorbitol, starch, sucrose, sugar spheres, talc, tribasic calcium phosphate, xylitol.

Examples of lubricants include calcium stearate, glyceryl monostearate, glyceryl palmitostearate, magnesium stearate, microcrystalline cellulose, sodium benzoate, sodium chloride, sodium lauryl sulphate, stearic acid, sodium stearyl fumarate, talc, zinc stearate.

Examples of glidants include colloidal silicon dioxide, powdered cellulose, magnesium trisilicate, silicon dioxide, talc.

Examples of disintegrants include alginic acid, carboxymethylcellulose calcium, carboxymethylcellulose sodium, colloidal silicon dioxide, croscarmellose sodium, crospovidone, guar gum, magnesium aluminium silicate, microcrystalline cellulose, methyl cellulose, polyvinylpyrrolidone, polacrilin potassium, pregelatinised starch, sodium alginate, sodium lauryl sulphate, sodium starch glycollate.

An example of a pharmaceutically acceptable wetting agent is sodium lauryl sulphate.

As required the solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice.

Compositions may, if desired, be in the form of a pack accompanied by written or printed instructions for use.

No adverse toxicological effects are expected for the compositions of the invention in the above mentioned dosage ranges.

### EXAMPLES

### Example 1, Delayed Release Composition

Delayed release can be achieved by coating tablets comprising 4mg or 8mg of Compound (I) as pure free base (pfb) with Eudragit L100-55, a gastric resistant polymer

The enteric coat consists of:

| | %w/w |
|---|---|
| Eudragit L30 D-55 (30% aqueous dispersion) | 76.8 |
| Triethyl Citrate | 7.7 |
| Talc Alphafil 500 | 15.5 |

### Example 2, Sustained Release by use of a matrix tablet

A matrix tablet is formed by tabletting the following mixture:

| | mg/tablet |
|---|---|
| Compound (I) | 8 (pfb) |
| Eudragit L100-55 | 150 |
| Lactose monohydrate | 50 |
| Eudragit RS powder | to 500 |

### Example 3, Sustained release by use of a semi-permeable membrane

The semi-permeable membrane consists of:

| | %w/w |
|---|---|
| Eudragit RS30D (30% aqueous dispersion) | 90 |
| Triethyl Citrate | 1 |
| Talc | 9 |

This membrane is applied to conventional tablets each comprising 4mg or 8mg of Compound (I) (pfb).

### Example 4, Sustained Release by use of a Mixed Eudragit matrix tablet

A matrix tablet is formed by tabletting the following mixture:

| | mg/tablet |
|---|---|
| Compound (I) | 8 (pfb) |
| Eudragit L100-55 | 74 |
| Eudragit RS powder | 18.5 |
| Colloidal Silicon dioxide | 0.6 |
| Magnesium stearate | 1.5 |
| Lactose monohydrate | to 150 |

### Example 5, Sustained Release by use of a Mixed Carbopol matrix tablet

A matrix tablet is formed by tabletting the following mixture:

| | mg/tablet |
|---|---|
| Compound (I) | 8 (pfb) |
| Anhydrous dibasic calcium phosphate | 35.7 |
| Carbopol 971P | 22.5 |
| Carbopol 974P | 7.5 |
| Talc | 0.75 |
| Lactose monohydrate | to 150 |

### Example 6, Delayed Release Composition

A capsule containing multiple pellet cores is formed using the following mixture:

| | mg/capsule |
|---|---|
| Compound (I) | 8 (pfb) |
| Microcrystalline cellulose | 133.5 |
| Lactose monohydrate | to 267 |

Delayed release can be achieved by coating the pellet cores with Eudragit L100-55, a gastric resistant polymer

The enteric coat consists of:

| | %w/w |
|---|---|
| Eudragit L30 D-55 (30% aqueous dispersion) | 76.8 |
| Triethyl Citrate | 7.7 |
| Talc Alphafil 500 | 15.5 |

### Example 7, Delayed Release Multiparticulate Composition

Delayed release multiparticulates are prepared by coating drug layered lactose spheres (the drug layer being 8mg of Compound (I) as pure free base (pfb) per dose) with either Eudragit L30D-55 or EudragitFS 30D, pH dependent polymers The drug layered multiparticulates are prepared by fluid-bed coating of the sphere with the required amount of Compound. (I), The drug layered multiparticulates therefore consist of:

| | %w/w |
|---|---|
| Compound (I)* | 5.40 |
| Opadry Clear | 3.0 |
| Polysorbate 80 (Tween 80) | 1.0 |
| Purfied Water | q.s. |
| Lactose spheres (25-30 mesh) | 200 mg |

| | |
|---|---|
| *This is based on Purity (as is) 99.2% w/w. Pure Free Base: 74.9% w/w. | |

The drug layered multiparticulates are then seal-coated with 2%, by weight, of film former Opadry Clear prior to application of the enteric coat,

The enteric, coat consists of:

| | %w/w |
|---|---|
| Eudragit L30 D-55 (30% aqueous dispersion) | 10-25 |
| Triethyl Citrate | 15* |
| Talc Alphafil 500 | 23.0* |
| Purified Water** | q.s. |

| | |
|---|---|
| * These percentages are based on the solid content of the Eudragit ** Sufficient water is added, such that the total solids content is 16%. | |

Or

| | |
|---|---|
| Eudragit FS 30D (30% aqueous dispersion) | 10-15 |
| Glyceryl Monostearate, NF | 3.0* |
| Triethyl Citrate | 1.0* |
| Purified Water** | q.s. |

| | |
|---|---|
| * These percentages are based on the solid content of the Eudragit ** Sufficient water is added such that the total solids content is 16%. | |

### Example 8, Sustained Release Multiparticulate Composition

Delayed release multiparticulates are prepared by coating drug layered lactose spheres (the drug layer being 8mg of Compound (I) as pure free base (pfb) per dose) with ethylcellulose polymer (Surelease).

The drug layered multiparticulates consist of:

| | % w/w |
|---|---|
| Compound (I)* | 5.40 |
| Opadry Clear | 3.0 |
| Polysorbate 80 (Tween 80) | 1.0 |
| Purfied Water | q.s. |
| Lactose spheres (25-30 mesh) | 200 mg |

| | |
|---|---|
| *This is based on Purity (as is) 99.2% w/w. Pure Free Base: 74.9% w/w. | |

The drug layered multiparticulates are seal coated with 2%, by weight, with film former Opadry Clear prior to the application of the semipermeable membrane.

The semipermeable membrane consists of:

| | %w/w |
|---|---|
| Surelease Clear | 10-20 |
| Purified Water* | q.s. |

| | |
|---|---|
| * Sufficient water is added such that the total solids content is 15%. | |

The multiparticulates can be admixed and filled into capsules or compressed into tablets to provide the desired release profile.

## Claims

1. A pharmaceutical composition which composition comprises an insulin sensitiser and a pharmaceutically acceptable carrier therefor, wherein the composition is arranged to provide a modified release of the insulin sensitiser.

2. A modified release pharmaceutical composition, which composition comprises an insulin sensitiser and a pharmaceutically acceptable carrier therefor, wherein the carrier is arranged to provide a modified release of the insulin sensitiser.

3. A composition according to claim 1 or claim 2, wherein the modified release is delayed, pulsed or sustained release.

4. A composition according to any one of claims 1 to 3, wherein the modified release is a delayed release.

5. A composition according to claim 4, wherein the composition is in the form of an enteric tablet formulation.

6. A composition according to claim 5, wherein the enteric coated tablet is a single layer tablet.

7. A composition according to claim 7, wherein the enteric coated tablet is a multi-layer tablet.

8. A composition according to any one of claims 5 to 7, wherein the tablet is coated with a gastric resistant polymer.

9. A composition according to claim 8, wherein the gastric resistant polymer is selected from the list consisting of Eudragit L100-55, methacrylates, cellulose acetate phthalate, polyvinyl acetate phthalate, hydroxypropyl methylcellulose phtahlate, in particular, Aquateric, Sureteric and HPMCP-HP-SSS.

10. A composition according to any one of claims 1 to 3, wherein the modified release is a sustained release.

11. A composition according to any one of claims 1 to 3, wherein the sustained release is provided by a sustained release matrix selected from disintegrating, non-disintegrating and eroding matrices.

12. A composition according to claim 11, wherein the disintegrating matrix tablet formulation is provided by incorporating methacrylates, methylcellulose and Methocel K4M into the matrix.

13. A composition according to claim 11, wherein the non disintegrating matrix tablet formulation is provided by incorporating Eudragit RS, methacrylates, cellulose acetates, hydroxypropyl methylcellulose phthalate, Carbopol 971P or HPMCP-HP-55S into the matrix.

14. A composition according to any one of claims 1 to 13, wherein the insulin sensitiser is Compound (I) or a derivative thereof.

15. A composition according to any one of claims 1 to 13, wherein the insulin sensitiser is 5-[4-[2-(N-methyl-N-(2-pyridyl)amino)ethoxy]benzyl]thiazolidine-2,4-dione, 5-[4-[2-(5-ethylpyridin-2-yl)ethoxy]benzyl] thiazolidine-2,4-dione (pioglitazone) or (+) -5-[[4-[(3,4-dihydro-6-hydroxy-2, 5, 7, 8-tetramethyl-2H-1-benzopyran-2-yl)methoxy]phenyl]methyl]-2,4-thiazolidinedione (troglitazone); or a derivative thereof.

16. A process for preparing a pharmaceutical composition, which composition comprises an insulin sensitiser and a pharmaceutically acceptable carrier therefor, which process comprises formulating the insulin sensitiser and the pharmaceutically acceptable carrier so as to enable a modified release of the insulin sensitiser.

17. A process for preparing a modified release pharmaceutical composition, which composition comprises an insulin sensitiser and a pharmaceutically acceptable carrier therefor, which process comprises formulating the insulin sensitiser and the pharmaceutically acceptable carrier, wherein the carrier is arranged so as to enable a modified release of the insulin sensitiser.
